# EUROPEAN PATENT APPLICATION

(11) **EP 3 909 675 A1**
(43) Date of publication of application: **17.11.2021**
(21) Application number: 20173869.7
(22) Date of filing: 11.05.2020
(51) Int. Cl.: B01J 29/18, B01J 27/24, B01J 31/02, C07C 1/24

(54) **PROCESS AND CATALYST FOR THE PREPARATION OF ETHYLENE**

(71) Applicant: BP P.L.C., London SW1Y 4PD (GB)
(72) Inventor: BUDA, Corneliu, Naperville, IL 60563 (US); DENNIS-SMITHERS, Benjamin James, Hull, East Yorkshire HU12 8DS (GB); JACKSON, Fiona, Hull, East Yorkshire HU12 8DS (GB); SUNLEY, John Glenn, Hull, East Yorkshire HU12 8DS (GB)
(74) Representative: BP Global Patents

(57) **Abstract**

A process for the preparation of ethylene by the dehydration of ethanol in the presence of a zeolite catalyst having the MOR framework code, wherein the process is operated at a temperature in the range of from 100 °C to 300 °C, for example from 140 °C to 270 °C, such as from 150 °C to 250 °C, and wherein the zeolite catalyst having the MOR framework code has been modified by the adsorption of an optionally substituted pyridine compound.

## Description

This invention relates in general to a dehydration process and in particular to a process for the dehydration of ethanol to prepare ethylene in the presence of a modified zeolite catalyst.

Ethylene is a widely used chemical and is produced on an industrial scale around the world. Processes for the preparation of ethylene are known in the art; for example, the preparation of ethylene by steam or catalytic cracking of hydrocarbons, or the preparation of ethylene by dehydration of ethanol using alumina or acid catalysts are known in the art.

Applicant has now found that the use of a zeolite catalyst having the MOR framework code which has been modified by the adsorption of an optionally substituted pyridine compound can be used to prepare ethylene from ethanol with low levels of ethane in the product stream.

Accordingly, the present invention provides a process for the preparation of ethylene by the dehydration of ethanol in the presence of a zeolite catalyst having the MOR framework code, wherein the process is operated at a temperature in the range of from 100 °C to 300 °C, for example from 140 °C to 270 °C, such as from 150 °C to 250 °C, and wherein the zeolite catalyst having the MOR framework code has been modified by the adsorption of an optionally substituted pyridine compound.

Advantageously, the use of a zeolite catalyst having the MOR framework code which has been modified by the adsorption of an optionally substituted pyridine compound allow productivity to ethylene to be improved in dehydration reactions of ethanol which are carried out in the presence of aluminosilicate zeolite catalysts.

Thus, according to another embodiment, the present invention provides a catalyst composition comprising a zeolite catalyst having the MOR framework code that has been modified by the adsorption of an optionally substituted pyridine compound.

Also, according to the present invention there is provided a method of improving the productivity to ethylene in a process for dehydrating ethanol in the presence of an aluminosilicate zeolite catalyst, wherein the catalyst is a zeolite catalyst having the MOR framework code, and where said method involves modifying the zeolite catalyst by the adsorption of an optionally substituted pyridine compound.

Yet further according to the present invention there is provided the use of an optionally substituted pyridine compound in a process for the catalytic dehydration of ethanol to ethylene to improve selectivity to ethylene, wherein the catalyst is a zeolite catalyst having the MOR framework code and the optionally substituted pyridine compound is adsorbed on to the zeolite catalyst having the MOR framework code.

A further aspect of the present invention provides a process for dehydrating ethanol to ethylene in the presence of a catalyst, wherein the catalyst is a zeolite catalyst having the MOR framework code, and wherein prior to using the catalyst in the dehydration process, the catalyst has been impregnated with an optionally substituted pyridine.

A further aspect of the present invention provides a process for dehydrating ethanol to ethylene in the presence of a catalyst, wherein the catalyst is a zeolite catalyst having the MOR framework code, and an optionally substituted pyridine compound is co-fed with the ethanol feed to the dehydration reaction, and wherein and the molar ratio of optionally substituted pyridine compound to ethanol is maintained at less than 1.

The catalytic dehydration reaction of ethanol to ethylene can be represented by the following equation:

Ethanol ⇄ Ethylene + Water

The dehydration of ethanol to ethylene can proceed directly, or alternatively and/or simultaneously, the dehydration reaction may also occur via an ether intermediate which can be represented by the following equations:

2 Ethanol ⇄ Diethyl ether + Water

Diethyl ether ⇄ Ethylene + Ethanol

In the present invention, the dehydration process is carried out in the presence of a zeolite catalyst having the MOR framework code. Mordenite is an aluminosilicate zeolite; aluminosilicate zeolites are crystalline microporous materials which have framework structures constructed from tetrahedra of SiO₄ and AlO₄ that share vertices. Such tetrahedral species are generally referred to as TO₄ species wherein the T atom is silicon or aluminium. Aluminium 'T' atoms can be partially or wholly replaced by one or more gallium, boron or iron atoms. For the purposes of the present invention, such gallium, boron or iron modified zeolites are considered to fall within the definition of the term 'aluminosilicate zeolites'.

A zeolite framework topology contains a regular array of pores, channels and/or pockets that vary in size, shape and dimensionality. These framework topologies or structure types of zeolites are assigned three-letter structure codes by the Structure Commission of the International Zeolite Association, under the authority of IUPAC.

A description of zeolites, their framework codes, structure, dimensionality, properties and methods of synthesis can be found in The Atlas of Zeolite Framework Types (C. Baerlocher, W. M. Meier, D. H. Olson, 5th Ed. Elsevier, Amsterdam, 2001) in conjunction with the web-based version (http://www.iza-structure.org/databases/).

Zeolite crystals contain pore or channel systems of molecular dimensions with fixed geometry and size and can be classified according to the number of channels running in different directions within the zeolite framework structure. A zeolite is described as 1-dimensional, 2-dimensional or 3-dimensional if the zeolite has one, two or three channels in different directions, respectively.

Zeolites may also be classified according to the size of their pores. Zeolite channels with pore openings limited by 8 T atoms in tetrahedral co-ordination are defined as having an 8-membered ring, zeolite channels with pore openings limited by 10 T atoms in tetrahedral co-ordination are defined as having a 10-membered ring, and zeolite channels with pore openings limited by 8 T atoms in tetrahedral co-ordination are defined as having a 12-membered ring. Zeolites can also conveniently be classified based upon the channel containing the largest pore opening, and zeolites with the largest pore openings limited by 8 T atoms in tetrahedral co-ordination (8-membered rings) may be defined as "small pore zeolites" (8-membered rings); zeolites with the largest pore openings limited by 10 T atoms in tetrahedral co-ordination (10-membered rings) may be defined as "medium pore zeolites"; and, zeolites with the largest pore openings limited by 12 T atoms in tetrahedral co-ordination (12-membered rings) may be defined as "large pore zeolites".

The zeolite framework code MOR is a large pore zeolite having a 1-dimensional structure. Examples of zeolites having framework type MOR include mordenite. In a specific embodiment of the present invention, the zeolite catalyst having the MOR framework code used in the present invention is mordenite.

Typically, zeolites are synthesised from synthesis mixtures comprising a silica source, an alumina source, alkali metal hydroxide and water in desired proportions. The synthesis mixture is maintained, with or without agitation, under temperature, pressure and time conditions sufficient to form a crystalline aluminosilicate zeolite. The resulting zeolite contains alkali metal as a cation. Such cations may be replaced by known ion-exchange techniques. For example, the zeolite may be contacted with aqueous solutions of ammonium salts to substitute ammonium ions for the alkali metal cations. Ammonium-form zeolites are also available commercially.

Whilst zeolites in their ammonium-form can be catalytically active, for use in the present invention it is preferred to utilise a zeolite in its hydrogen-form (H-form). H-form zeolites are commercially available. Alternatively, an ammonium-form zeolite can be fully or partially converted to the H-form by known techniques, for example by calcining the ammonium-form zeolite, in air or inert gas, at high temperature, for example at a temperature of 500 °C or higher.

In some or all embodiments of the present invention, the zeolite catalyst having the MOR framework code is a zeolite which is a hydrogen-form (H-form) zeolite.

Organic structure directing agents (OSDA) may also be used in the synthesis of mordenite. In such syntheses, the mordenite may be synthesised by the crystallisation of a zeolite from a synthesis mixture comprising a source of silica, a source of alumina, a source of alkali or alkaline earth metal, water and an organic structure directing agent. Synthesis of zeolites using organic structure directing agents is known in the art, and methods of synthesising mordenite using OSDA are described in WO2014/135662, which is incorporated herein by reference. When an OSDA is used in the synthesis of mordenite, the OSDA may optionally be removed before using the mordenite in the present invention, for example by calcining at high temperature.

For use in the present invention, the zeolite catalyst having the MOR framework code may be composited with at least one binder material. The binder material may be a refractory inorganic oxide, such as silicas, aluminas, alumina-silicates, magnesium silicates, magnesium aluminium silicates, titanias and zirconias.

For use in the present invention, the relative proportions of the zeolite catalyst having the MOR framework code and binder material in the composite may vary widely. Suitably, the binder material can be present in an amount of from 10% to 90% by weight of the composite.

For use in the present invention, the silica to alumina molar ratio of the zeolite catalyst having the MOR framework code may vary widely but suitably is in the range 10 to 300, for example in the range 20 to 280, such as in the range 20 to 100.

In some or all embodiments of the present invention, the optionally substituted pyridine compound may be compounds of formula I below:

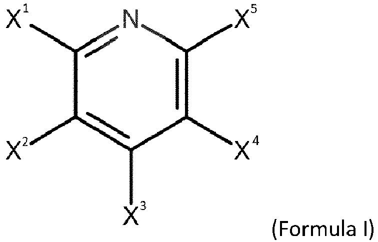

Wherein each of X¹ to X⁵ are independently selected from hydrogen, hydroxy groups, optionally substituted hydrocarbyl groups, optionally substituted alkoxy groups, optionally substituted aromatic groups, or any two of X¹ to X⁵ may be bonded together with an optionally substituted hydrocarbyl group or an optionally substituted alkoxy group to form a cyclic group.

By the term substituted hydrocarbyl as used herein, it is meant a hydrocarbyl component which comprises one or more heteroatoms. The one or more heteroatoms may conveniently be independently selected from nitrogen, oxygen, or a halide. By the term substituted alkoxy as used herein, it is meant an alkoxy component which comprises one or more heteroatoms. The one or more heteroatoms may conveniently be independently selected from nitrogen, oxygen, or a halide. By the term substituted aromatic as used herein, it is meant an aromatic component which comprises one or more heteroatoms. The one or more heteroatoms may conveniently be independently selected from nitrogen, oxygen, or a halide.

In the embodiments wherein one or more of the X¹ to X⁵ groups is an optionally substituted hydrocarbyl group, the hydrocarbyl group is preferably selected from an optionally substituted C₁-C₁₁ hydrocarbyl group, preferably a non-substituted C₁-C₁₁ hydrocarbyl group. The C₁-C₁₁ hydrocarbyl group may be linear or branched, or may contain a cyclic group comprising three or more carbon atoms. In some or all embodiments wherein one or more of the X¹ to X⁵ groups is an optionally substituted hydrocarbyl group, the hydrocarbyl group is selected from a linear or branched C₁-C₁₁ alkyl group; for example, the hydrocarbyl group may be selected from a linear or branched C₁-C₉ alkyl group, or a linear or branched C₁-C₇ alkyl group. Non-limiting examples of suitable hydrocarbyl groups include methyl, ethyl, n-propyl, i-propyl, n-butyl, i-butyl, tert-butyl, n-pentyl, n-hexyl, n-heptyl or n-octyl alkyl group.

In the embodiments wherein one or more of the X¹ to X⁵ groups is an optionally substituted alkoxy group, the alkoxy group is preferably selected from an optionally substituted C₁-C₁₁ alkoxy group, preferably a non-substituted C₁-C₁₁ alkoxy group. The C₁-C₁₁ alkoxy group may be linear or branched, or may contain a cyclic group comprising three or more carbon atoms. In some or all embodiments wherein one or more of the X¹ to X⁵ groups is an optionally substituted alkoxy group, the hydrocarbyl group is selected from a linear or branched C₁-C₁₁ alkoxy group; for example, the alkoxy group may be selected from a linear or branched C₁-C₉ alkoxy group, or a linear or branched C₁-C₇ alkoxy group. Non-limiting examples of suitable alkoxy groups include methoxy, ethoxy, n-propoxy, i-propoxy, n-butoxy, i-butoxy, tert-butoxy, n-pentoxy, n-hexoxy, n-heptoxy or n-octoxy groups.

In the embodiments wherein one or more of the X¹ to X⁵ groups is an optionally substituted aromatic group, the aromatic group is preferably selected from an optionally substituted phenyl group. The optionally substituted phenyl group may be a hydrocarbyl substituted phenyl group; the hydrocarbyl group substituent on the aromatic group may be a linear or branched C₁-C₁₁ hydrocarbyl group, or may contain a cyclic group comprising three or more carbon atoms. In some or all embodiments wherein one or more of the X¹ to X⁵ groups is a hydrocarbyl substituted phenyl group, the hydrocarbyl group is selected from a linear or branched C₁-C₁₁ alkyl group; for example, the hydrocarbyl group may be selected from a linear or branched C₁-C₉ alkyl group, or a linear or branched C₁-C₇ alkyl group. Non-limiting examples of suitable hydrocarbyl groups include methyl, ethyl, n-propyl, i-propyl, n-butyl, i-butyl, tert-butyl, n-pentyl, n-hexyl, n-heptyl or n-octyl alkyl groups.

In some or all embodiments of the present invention, the optionally substituted pyridine compound may be compounds of formula I, wherein each of X¹ to X⁵ are independently selected from hydrogen, C₁-C₁₁ hydrocarbyl groups, C₁-C₁₁ alkoxy groups.

In some or all embodiments of the present invention, the optionally substituted pyridine compound may be compounds of formula I, wherein each of X¹ to X⁵ are independently selected from hydrogen, C₁-C₉ hydrocarbyl groups, C₁-C₉ alkoxy groups.

In some or all embodiments of the present invention, the optionally substituted pyridine compound may be compounds of formula I, wherein each of X¹ to X⁵ are independently selected from hydrogen, C₁-C₇ hydrocarbyl groups, C₁-C₇ alkoxy groups.

In some or all embodiments of the present invention, the optionally substituted pyridine compound may be compounds of formula I, wherein all of the X¹ to X⁵ are hydrogen or, or wherein one of the X¹ to X⁵ groups is a group selected from C₁-C₁₁ hydrocarbyl groups, C₁-C₁₁ alkoxy groups, and the other the X¹ to X⁵ groups are all hydrogen groups.

In some or all embodiments of the present invention, the optionally substituted pyridine compound may be compounds of formula I, wherein all of the X¹ to X⁵ are hydrogen or, or wherein one of the X¹ to X⁵ groups is a group selected from C₁-C₉ hydrocarbyl groups, C₁-C₉ alkoxy groups, and the other the X¹ to X⁵ groups are all hydrogen groups.

In some or all embodiments of the present invention, the optionally substituted pyridine compound may be compounds of formula I, wherein all of the X¹ to X⁵ are hydrogen or, or wherein one of the X¹ to X⁵ groups is a group selected from C₁-C₇ hydrocarbyl groups, C₁-C₇ alkoxy groups, and the other the X¹ to X⁵ groups are all hydrogen groups.

Non-limiting examples of suitable optionally substituted pyridine compounds which may be used in the present invention include pyridine, 2-methylpyridine, 3-methylpyridine, 4-methylpyridine, 2-ethylpyridine, 3-ethylpyridine, 4-ethylpyridine, 4-n-propylpyridine, 3-n-butylpyridine, 4-hydroxypyridine, 4-methoxypyridine, 4-ethoxypyridine, collidines, and lutidines; particularly suitable examples include pyridine, 4-methoxypyridine, and 3-ⁿbutylpyridine.

The optionally substituted pyridine compounds may be used individually, or mixture of any two or more of the optionally substituted pyridine compounds may also be used in the present invention. In some or all embodiments of the present invention, only a single species of optionally substituted pyridine compounds is used.

In some or all embodiments of the present invention, the optionally substituted pyridine compound is adsorbed on to the zeolite catalyst having the MOR framework code by impregnation. The method of impregnation is not limited and any technique known in the art may be used, for example, incipient wetness technique, excess solution technique, or vapour phase impregnation.

The optionally substituted pyridine compound may be used as the impregnation solution (or vapour) directly, or a dilute solution (or vapour) of the optionally substituted pyridine compound may be used. When a dilute solution of the optionally substituted pyridine compound is used, the solvent for the impregnation solution may suitably be an aqueous solution, an organic solution, or a mixture of aqueous and organic solvent(s), depending upon the solubility of the optionally substituted pyridine compound being used; non-limiting examples of suitable solvents include water, alcohols, for example methanol or ethanol, ethers, for example diethyl ether, and mixtures thereof, such as aqueous alcoholic solutions, for example an aqueous methanol or ethanol solution.

Preferably, the optionally substituted pyridine compound is adsorbed on to the zeolite catalyst having the MOR framework code prior to using the catalyst in the dehydration process.

Additionally and/or alternatively, the optionally substituted pyridine compound may be added as a component of the feed to the dehydration process.

In the embodiments of the present invention wherein the optionally substituted pyridine compound is added as a component of the feed to the dehydration process, the optionally substituted pyridine compound may be added upon first introduction of the feed, added periodically to the feed, or may be added continuously to the feed. If the optionally substituted pyridine compound is added upon first introduction of the feed to the dehydration reaction, the initial operating temperature of the dehydration reaction is preferably maintained below 200 °C, for example at or below 150 °C, during the period in which the optionally substituted pyridine compound is added.

In the embodiments of the invention where the optionally substituted pyridine compound is added continuously to the feed, the molar ratio of optionally substituted pyridine compound to ethanol is maintained throughout the dehydration reaction at less than 1. By the term 'molar ratio of optionally substituted pyridine compound to ethanol' or the like, it is meant the molar ratio the total amount of ethanol present in the dehydration reaction to the total amount of optionally substituted pyridine compound compounds in the dehydration reaction, i.e. (total amount of ethanol) : (total amount of optionally substituted pyridine compound). In the embodiments of the invention where the optionally substituted pyridine compound is added continuously to the feed, the molar ratio of optionally substituted pyridine compound to ethanol is maintained in the range 0.000000001 : 1 to less than 0.5 : 1, preferably in the range of 0.00000005 : 1 to less than 0.5 : 1. In some or all embodiments of the present invention, the molar ratio of optionally substituted pyridine compound to ethanol is maintained in the range of 0.0000001 : 1 to less than 0.5 : 1, for example 0.0000005 : 1 to 0.2 : 1, such as 0.00001 : 1 to 0.2 : 1.

In some or all embodiments of the present invention, the ethanol feed to the dehydration does not comprise any optionally substituted pyridine compound added thereto.

The dehydration process of the present invention may be part of a co-production process where the ethylene is further reacted to form products within the same reaction system, or it may be a standalone process. In some or all embodiments of the present invention, the dehydration process is a standalone process.

The ethanol feed to the dehydration process in the present invention is not limited.

The dehydration of ethanol to ethylene of the present invention produces ethylene with low concentrations of ethane by-product. The dehydration of ethanol to ethylene of the present invention also produces water as a by-product, and typically also produces a quantity of diethylether and some unconverted ethanol. Advantageously, any diethyl ether produced in the dehydration process of the present invention may be converted to ethylene through an elimination reaction. In some or all embodiments of the dehydration process of the present invention, at least part of any unconverted ethanol together with any diethyl ether and optionally the by-product water present in the product stream may be separated from the ethylene product and recycled back to the dehydration process.

In some or all embodiments of the present invention, the ethanol feed to the dehydration process comprises at least 50 %wt. ethanol, for example at least 60 %wt. ethanol, such as at least 70 %wt. ethanol. Other components that may be present in the ethanol feed typically include water, diethyl ether, and nitrogen containing compounds.

In some or all embodiments of the present invention, the fresh ethanol feed to the dehydration process (i.e. the ethanol feed excluding any recycle streams) comprises at least 80 %wt. ethanol, for example at least 90 %wt. ethanol, such as at least 95 %wt. ethanol. Other components that may be present in the fresh ethanol feed typically include water and nitrogen containing compounds.

In some or all embodiments of the present invention, the ethanol feed to the dehydration process comprises bioethanol (i.e. ethanol that has been prepared from the fermentation of suitable biomass). Without wishing to be bound by theory, it is believed that the presence of water does not have an adverse effect on the dehydration process of the present invention, therefore, the dehydration process of the present invention may be performed using azeotropic ethanol-water mixture which has been obtained from the distillation of a source of ethanol prepared from the fermentation of suitable biomass.

The dehydration process is carried out as a heterogeneous process, preferably as a vapour phase heterogeneous process. The type of reactor used for the dehydration process is not limited, and it may be suitably carried out in any type of reactor within which a heterogeneous process may be performed. Non-limiting types of reactors with which the dehydration reaction may be performed include tank reactors, multi-tubular reactors, plug-flow reactors, loop reactors, fluidized bed reactors, reactive distillation columns, and any other suitable reactor which comprises a heat transfer surface.

The dehydration process may be carried out at a temperature of from 100 to 300 °C. In some or all embodiments of the present invention, the dehydration process is carried out at a temperature of from 140 to 270 °C, for example from 150 to 250 °C.

Suitably, the dehydration process may be carried out at atmospheric pressure or at elevated pressure. In some or all embodiments of the present invention, the dehydration process is carried out at a total pressure of atmospheric pressure to 3000kPa.

The gas hourly space velocity (GHSV) at which the dehydration process of the present invention is carried out is not particularly limited. In some or all embodiments of the present invention, the dehydration process is carried out at a total gas hourly space velocity (GHSV) in the range 500 to 40,000 h⁻¹.

The dehydration process may be carried out using one or more beds of the zeolite catalyst.

The dehydration process may be operated as either a continuous or a batch process, preferably as a continuous process.

The dehydration process generates a crude reaction product comprising ethylene, water, unconverted ethanol, and typically diethyl ether. One or more components of the crude reaction product may be recycled as feed to the process.

The ethylene product has a lower boiling point than ethanol, water, and diethyl ether, and as such may be readily separated from the crude reaction product by any suitable method known in the art.

The dehydration process of the present invention provides a process for the dehydration of ethanol to ethylene with a high selectivity to ethylene. Advantageously, the dehydration process of the present invention has been found to prepare an ethylene product stream with low levels of ethane. Additionally, it has been observed that the dehydration process of the present invention results in a product stream contain lower levels of diethyl ether compared to the use of zeolites which have not been modified by the adsorption of an optionally substituted pyridine compound. It has further been observed that the dehydration process of the present invention results in a product stream contain lower levels of higher (C3+) olefins and alkanes compared to the use of zeolites which have not been modified by the adsorption of an optionally substituted pyridine compound. It has further been observed that the dehydration process of the present invention results in a product stream contain lower levels of acetaldehyde compared to the use of zeolites which have not been modified by the adsorption of an optionally substituted pyridine compound.

Without wishing to be bound by theory, it is believed that the suppression of the production of diethyl ether, which is an exothermic reaction, reduces any temperature increases in the catalyst bed and therefore improves the catalyst stability can lead to a reduction in by-products, and can facilitate the use of higher temperatures without resulting in an unacceptable increase in by-product formation or reduction in catalyst life.

Another embodiment of the present invention provides a catalyst composition comprising a zeolite catalyst having the MOR framework code that has been modified by the adsorption of an optionally substituted pyridine compound.

Another embodiment of the present invention provides a method of improving the productivity to ethylene in a process for dehydrating ethanol in the presence of an aluminosilicate zeolite catalyst, wherein the catalyst is a zeolite catalyst having the MOR framework code, and where said method involves modifying the zeolite catalyst by the adsorption of an optionally substituted pyridine compound.

Another embodiment of the present invention provides the use of an optionally substituted pyridine compound in a process for the catalytic dehydration of ethanol to ethylene to improve selectivity to ethylene, wherein the catalyst is a zeolite catalyst having the MOR framework code and the optionally substituted pyridine compound is adsorbed on to the zeolite catalyst having the MOR framework code.

The invention is now illustrated with reference to the following non-limiting Examples.

### Examples

### Preparation of Catalyst A

NH₄-mordenite (CBV21A, Zeolyst International) was calcined in air at 500 °C for 4 hours to convert it into the H-form.

### Preparation of Catalyst B

2-ethylpyridine (0.14 mL) was dissolved in methanol (25 mL) before being added to Catalyst A (2 g) and the mixture heated to reflux (∼ 65 °C) for 72 hours whilst stirring. The mixture was then allowed to cool before being filtered and the catalyst residue washed with methanol (3 x 25 mL). The catalyst residue was then dried *in vacuo* for 24 hours to afford a white solid of 2-ethylpyridine doped H-mordenite.

### Preparation of Catalyst C

3-ethylpyridine (0.14 mL) was dissolved in methanol (25 mL) before being added to Catalyst A (2 g) and the mixture heated to reflux (∼ 65 °C) for 72 hours whilst stirring. The mixture was then allowed to cool before being filtered and the catalyst residue washed with methanol (3 x 25 mL). The catalyst residue was then dried *in vacuo* for 24 hours to afford a white solid of 3-ethylpyridine doped H-mordenite.

### Preparation of Catalyst D

4-ethylpyridine (0.14 mL) was dissolved in methanol (25 mL) before being added to Catalyst A and the mixture heated to reflux (∼ 65 °C) for 72 hours whilst stirring. The mixture was then allowed to cool before being filtered and the catalyst residue washed with methanol (3 x 25 mL). The catalyst residue was then dried *in vacuo* for 24 hours to afford a white solid of 4-ethylpyridine doped H-mordenite.

### Preparation of Catalyst E

4-hydroxypyridine (0.35 g) was dissolved in methanol (25 mL) before being added to Catalyst A (2 g) and the mixture heated to reflux (∼ 65 °C) for 72 hours whilst stirring. The reaction mixture was then allowed to cool before being filtered and the catalyst residue washed with methanol (3 x 25 mL). The catalyst residue was then dried *in vacuo* for 24 hours to afford a white solid of 4-hydroxypyridine doped H-mordenite.

### Preparation of Catalyst F

4-methoxypyridine (0.262 mL) was dissolved in methanol (25 mL) before being added to Catalyst A (2 g) and the mixture heated to reflux (∼ 65 °C) for 72 hours whilst stirring. The reaction mixture was then allowed to cool before being filtered and the catalyst residue washed with methanol (3 x 25 mL). The catalyst residue was then dried *in vacuo* for 24 hours to afford a white solid of 4-methoxypyridine doped H-mordenite.

### Preparation of Catalyst G

Pyridine (0.21 mL) was dissolved in methanol (25 mL) before being added to Catalyst A (2 g) and the mixture heated to reflux (∼ 65 °C) for 72 hours whilst stirring. The reaction mixture was then allowed to cool before being filtered and the catalyst residue washed with methanol (3 x 25 mL). The catalyst residue was then dried *in vacuo* for 24 hours to afford a white solid of pyridine doped H-mordenite.

### Preparation of Catalyst K

2.02g of Catalyst A was loaded into a tubular glassware reactor. Nitrogen was passed over the catalyst for 1 hour at 50 mL/min whilst at ambient temperature and pressure. The reactor was then heated to 150 °C at 5 °C/min. A gas feed of nitrogen was sparged though a saturator containing liquid pyridine at room temperature and then passed over the catalyst for 1.5 hours at 50 mL/min. A nitrogen only gas feed was then passed over the catalyst at 100 mL/min and the reactor held at 150 °C for 30 minutes. The temperature was then ramped to 450 °C at 5 °C/min and held for 24 hours. The heater was cooled to room temperature whilst nitrogen was passed over it at 50 mL/min and the catalyst removed.

### Preparation of Catalyst L - neat pyridine, no solvent

Pyridine (25 mL) was added to Catalyst A (2 g) and the mixture heated to reflux (∼ 65 °C) for 72 hours without stirring. The reaction mixture was then allowed to cool before being filtered and the catalyst residue was then dried *in vacuo* for 24 hours to afford a white solid of pyridine doped H-mordenite.

### Preparation of Catalyst M, neat 3-ⁿbutylpyridine, no solvent

3-ⁿbutylpyridine (25 mL) was added to Catalyst A (2 g) and the mixture heated to 80 °C for 72 hours whilst stirring. The reaction mixture was then allowed to cool before being filtered and the catalyst residue was then dried *in vacuo* for 48 hours to afford a white solid of 3-ⁿbutylpyridine doped H-mordenite.

### General Reaction Method and Apparatus

The ethanol dehydration reactions were carried out using a 16-channel parallel fixed-bed stainless steel reactor system. Each reactor housed a 25 mg bed of catalyst (having particle size fraction of 100 to 200 microns diameter) loaded on top of a 6 cm deep bed of an inert material (carborundum). The reactor volume above the catalyst was also packed with carborundum.

Each reactor (2 mm internal diameter) was heated to a temperature of 150 °C at the start of the reaction and was maintained at a total pressure of 1100 kPa throughout the reaction. A gaseous feed comprising 10 mol% ethanol and inert gas (8.18 mol% He and 81.82 mol% nitrogen) was introduced into the reactor at a temperature of 150 °C and allowed to flow through the catalyst bed. This total gaseous feed to each reactor was 133 mmol h⁻¹. After 24 hours at 150 °C, each reactor was then heated via a series of temperature steps (180 °C, 210 °C, 230 °C and 250 °C) to a final reaction temperature of 270 °C. A dwell time of approximately 24 hours was applied at each temperature step.

The effluent stream from each reactor was diluted with inert gas (nitrogen) and was periodically analysed by online gas chromatography to determine the yields of diethyl ether and ethylene products and ethane by-product.

**Table 1: Data at 210 °C reaction temperature**

| Catalyst | Dopant | TOS (h) | Catalyst mass (mg) | EtOH conversion % | Ethylene STYW (gkg_{cat}⁻¹h⁻¹) | DEE STYW (gkg_{cat}⁻¹h⁻¹) | Ethane in C2 hydrocarbons (ppm) | Acetaldehyde STYW (gkg_{cat}⁻¹h⁻¹) |
|---|---|---|---|---|---|---|---|---|
| A | None | 93.9 | 25 | 81.0 | 1434 | 14044 | 1554 | 0.014 |
| B | 2-Ethylpyridine | 92.6 | 25 | 13.9 | 1603 | 610 | 286 | 0.009 |
| C | 3-Ethylpyridine | 92.0 | 25 | 11.2 | 1478 | 282 | 250 | 0.009 |
| D | 4-Ethylpyridine | 93.8 | 25 | 13.4 | 1479 | 265 | 617 | 0.011 |
| E | 4-Hydroxypyridine | 92.8 | 25 | 8.3 | 972 | 88 | 0* | 0.007 |
| F | 4-Methoxypyridine | 93.6 | 25 | 8.3 | 1061 | 97 | 0* | 0.007 |
| G | Pyridine | 91.1 | 25 | 9.3 | 1149 | 340 | 158 | 0.007 |
| K | Pyridine (vapour phase impregnation) | 94.5 | 25 | 16.6 | 2026 | 418 | 175 | 0.005 |
| L | Pyridine (no solvent) | 93.2 | 25 | 10.9 | 1345 | 301 | 113 | 0.004 |
| M | 3-ⁿbutylpyridine (no solvent) | 92.8 | 25 | 5.8 | 814 | 0* | 0* | 0.004 |

| | | | | | | | | |
|---|---|---|---|---|---|---|---|---|
| *Below detection limit Space Time Yield (STY) is quoted in grammes of product per kilogramme of catalyst per hour, g kgcat⁻¹h⁻¹ DEE STYW detection limit: 0.15 gkg_{cat}⁻¹h⁻¹ Ethane in ethylene detection limit: 0.03 ppm Ethane make quoted on a mole ppm basis relative to the sum of moles of C2 hydrocarbons, ethylene plus ethane, produced | | | | | | | | |

**Table 2: Data at 230 °C reaction temperature**

| Catalyst | Dopant | TOS (h) | Catalyst mass (mg) | EtOH conversion % | Ethylene STYW (gkg_{cat}⁻¹h⁻¹) | DEE STYW (g kg_{cat}⁻¹h⁻¹) | Ethane in C2 hydrocarbons (ppm) | Acetaldehyde STYW (gkg_{cat}⁻¹h⁻¹) |
|---|---|---|---|---|---|---|---|---|
| A | None | 118.6 | 25 | 84.5 | 4345 | 10349 | 970 | 0.042 |
| B | 2-Ethylpyridine | 117.2 | 25 | 32.7 | 4189 | 411 | 775 | 0.025 |
| C | 3-Ethylpyridine | 116.7 | 25 | 33.1 | 4301 | 261 | 322 | 0.011 |
| D | 4-Ethylpyridine | 118.4 | 25 | 33.8 | 4338 | 228 | 607 | 0.023 |
| E | 4-Hydroxypyridine | 117.4 | 25 | 25.2 | 3148 | 143 | 81 | 0.018 |
| F | 4-Methoxypyridine | 118.2 | 25 | 26.2 | 3245 | 151 | 62 | 0.014 |
| G | Pyridine | 115.7 | 25 | 27.6 | 3406 | 479 | 107 | 0.011 |
| K | Pyridine (vapour phase impregnation) | 120 | 25 | 40.7 | 5365 | 446 | 207 | 0.005 |
| L | Pyridine (no solvent) | 118.3 | 25 | 29.9 | 3918 | 422 | 76 | 0.004 |
| M | 3-ⁿbutylpyridine (no solvent) | 117.8 | 25 | 11.2 | 1579 | 0* | 166 | 0.004 |

| | | | | | | | | |
|---|---|---|---|---|---|---|---|---|
| *Below detection limit Space Time Yield (STY) is quoted in grammes of product per kilogramme of catalyst per hour, g kgcat⁻¹h⁻¹ DEE STYW detection limit: 0.15 g kg_{cat}⁻¹h⁻¹ Ethane make quoted on a mole ppm basis relative to the sum of moles of C2 hydrocarbons, ethylene plus ethane, produced | | | | | | | | |

**Table 3: Data at 250 °C reaction temperature**

| Catalyst C no. | Catalyst | TOS (h) | Catalyst mass (mg) | EtOH conversion % | Ethylene STYW (gkg_{cat}⁻¹h⁻¹) | DEE STYW (g kg_{cat}⁻¹h⁻¹) | Ethane in C2 hydrocarbons (ppm) | Acetaldehyde STYW (gkg_{cat}⁻¹h⁻¹) |
|---|---|---|---|---|---|---|---|---|
| A | None | 143.1 | 25 | 88.6 | 9250 | 3945 | 1144 | 0.090 |
| B | 2-Ethylpyridine | 141.8 | 25 | 47.5 | 6474 | 116 | 981 | 0.030 |
| C | 3-Ethylpyridine | 141.2 | 25 | 64.9 | 8875 | 132 | 229 | 0.023 |
| D | 4-Ethylpyridine | 142.9 | 25 | 65.8 | 8852 | 132 | 466 | 0.021 |
| E | 4-Hydroxypyridine | 141.9 | 25 | 52.6 | 7174 | 126 | 69 | 0.018 |
| F | 4-Methoxypyridine | 142.7 | 25 | 51.7 | 6940 | 131 | 53 | 0.016 |
| G | Pyridine | 143.3 | 25 | 58.0 | 7451 | 490 | 75 | 0.018 |
| K | Pyridine (vapour phase impregnation) | 144.3 | 25 | 74.7 | 10201 | 209 | 183 | 0.011 |
| L | Pyridine (no solvent) | 143.0 | 25 | 63.9 | 8715 | 445 | 89 | 0.005 |
| M | 3-ⁿbutylpyridine (no solvent) | 142.4 | 25 | 17.3 | 2397 | 0* | 178 | 0.005 |

| | | | | | | | | |
|---|---|---|---|---|---|---|---|---|
| *Below detection limit Space Time Yield (STY) is quoted in grammes of product per kilogramme of catalyst per hour, g kgcat⁻¹h¹ DEE STYW detection limit: 0.15 g kg_{cat}⁻¹h⁻¹ Ethane make quoted on a mole ppm basis relative to the sum of moles of C2 hydrocarbons, ethylene plus ethane, produced | | | | | | | | |

**Table 4: Data at 270 °C reaction temperature**

| Catalyst C no. | Catalyst | TOS (h) | Catalyst mass (mg) | EtOH conversion % | Ethylene STYW (gkg_{cat}⁻¹h⁻¹) | DEE STYW (g kg_{cat}⁻¹h⁻¹) | Ethane in C2 hydrocarbons (ppm) | Acetaldehyde STYW (gkg_{cat}⁻¹h⁻¹) |
|---|---|---|---|---|---|---|---|---|
| A | None | 167.6 | 25 | 96.9 | 10799 | 3112 | 4273 | 0.123 |
| B | 2-Ethylpyridine | 166.3 | 25 | 45.5 | 6057 | 39 | 241 | 0.023 |
| C | 3-Ethylpyridine | 165.7 | 25 | 91.9 | 12488 | 66 | 147 | 0.033 |
| D | 4-Ethylpyridine | 167.4 | 25 | 91.2 | 12237 | 60 | 140 | 0.030 |
| E | 4-Hydroxypyridine | 166.5 | 25 | 79.0 | 11009 | 59 | 75 | 0.026 |
| F | 4-Methoxypyridine | 167.2 | 25 | 74.4 | 10036 | 167 | 78 | 0.023 |
| G | Pyridine | 167.8 | 25 | 89.7 | 11755 | 334 | 75 | 0.025 |
| K | Pyridine (vapour phase impregnation) | 191.2 | 25 | 95.6 | 13174 | 76 | 152 | 0.019 |
| L | Pyridine (no solvent) | 189.9 | 25 | 94.2 | 13085 | 257 | 102 | 0.011 |

| | | | | | | | | |
|---|---|---|---|---|---|---|---|---|
| Space Time Yield (STY) is quoted in grammes of product per kilogramme of catalyst per hour, g kgcat⁻¹h⁻¹ Ethane make quoted on a mole ppm basis relative to the sum of moles of C2 hydrocarbons, ethylene plus ethane, produced | | | | | | | | |

**Table 5: Analytical information for catalysts**

| **Catalyst** | **Dopant** | **C wt.%** | **N wt.%** |
|---|---|---|---|
| A | None | 0* | 0.3 |
| B | 2-Ethylpyridine | 8.0 | 1.5 |
| C | 3-Ethylpyridine | 11.3 | 1.0 |
| D | 4-Ethylpyridine | 5.3 | 0.9 |
| E | 4-Hydroxypyridine | 5.6 | 1.3 |
| F | 4-Methoxypyridine | 6.6 | 1.3 |
| G | Pyridine | 5.9 | 1.0 |
| K | Pyridine (vapour phase impregnation) | 3.8 | 1.0 |
| L | Pyridine (no solvent) | 6.7 | 1.7 |
| M | 3-ⁿbutylpyridine (no solvent) | 10.1 | 1.5 |

| | | | |
|---|---|---|---|
| *Below detection limit; detection limit: <0.1 wt.% for C, H and N. | | | |

## Claims

1. A process for the preparation of ethylene by the dehydration of ethanol in the presence of a zeolite catalyst having the MOR framework code, wherein the process is operated at a temperature in the range of from 100 °C to 300 °C, for example from 140 °C to 270 °C, such as from 150 °C to 250 °C, and wherein the zeolite catalyst having the MOR framework code has been modified by the adsorption of an optionally substituted pyridine compound.

2. A process according to Claim 1, wherein the process is operated in the vapour phase.

3. A process according to Claim 1 or Claim 2, wherein the optionally substituted pyridine compound is a compound of formula I: 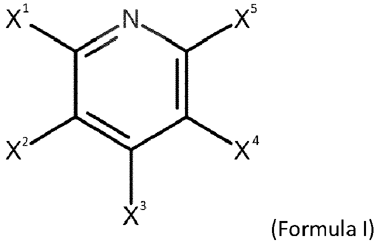 wherein each of X¹ to X⁵ are independently selected from hydrogen, hydroxy groups, optionally substituted hydrocarbyl groups, optionally substituted alkoxy groups, optionally substituted aromatic groups, or any two of X¹ to X⁵ may be bonded together with an optionally substituted hydrocarbyl group or an optionally substituted alkoxy group to form a cyclic group.

4. A process according to Claim 3, wherein each of X¹ to X⁵ are independently selected from hydrogen, C₁-C₁₁ hydrocarbyl groups, C₁-C₁₁ alkoxy groups.

5. A process according to Claim 3, wherein one of the X¹ to X⁵ groups is a group selected from C₁-C₁₁ hydrocarbyl groups, C₁-C₁₁ alkoxy groups, and the other the X1 to X5 groups are all hydrogen groups.

6. A process according to Claim 1 or Claim 2, wherein the optionally substituted pyridine compound is selected from pyridine, 2-methylpyridine, 3-methylpyridine, 4-methylpyridine, 2-ethylpyridine, 3-ethylpyridine, 4-ethylpyridine, 4-n-propylpyridine, 3-n-butylpyridine, 4-hydroxypyridine, 4-methoxypyridine, 4-ethoxypyridine, collidines, and lutidines.

7. A process according to any one of Claims 1 to 6, wherein the zeolite catalyst having the MOR framework code is mordenite.

8. A process according to Claim 7, wherein the mordenite has been synthesised using an organic-structure directing agent (OSDA)

9. A process according to any one of Claims 1 to 8, wherein the zeolite catalyst having the MOR framework code is composited with a binder material.

10. A process according to any one of Claims 1 to 9, wherein the optionally substituted pyridine compound is adsorbed on to the zeolite catalyst having the MOR framework code by impregnation prior to using the catalyst in the dehydration process.

11. A process according to any one of Claims 1 to 10, wherein the optionally substituted pyridine compound is added as a component of the feed to the dehydration process.

12. A process according to any one of Claims 1 to 11, wherein at least part of any unconverted ethanol together with any diethyl ether and optionally the by-product water present in the product stream is separated from the ethylene product and recycled back to the dehydration process.

13. A process according to any one of Claims 1 to 12, wherein the ethanol feed to the dehydration process comprises at least 50 %wt. ethanol.

14. A process according to any one of Claims 1 to 15, wherein the ethanol feed to the dehydration process comprises bioethanol.

15. A catalyst composition comprising a zeolite catalyst having the MOR framework code that has been modified by the adsorption of an optionally substituted pyridine compound.

16. A catalyst composition according to Claim 15, wherein the zeolite catalyst having the MOR framework code is mordenite.

17. A catalyst composition according to Claim 16, wherein the mordenite has been synthesised using an organic-structure directing agent (OSDA).

18. Use of an optionally substituted pyridine compound in a process for the catalytic dehydration of ethanol to ethylene to improve selectivity to ethylene, wherein the catalyst is a zeolite catalyst having the MOR framework code and the optionally substituted pyridine compound is adsorbed on to the zeolite catalyst having the MOR framework code.

19. A method of improving the productivity to ethylene in a process for dehydrating ethanol in the presence of an aluminosilicate zeolite catalyst, wherein the catalyst is a zeolite catalyst having the MOR framework code, and where said method involves modifying the zeolite catalyst by the adsorption of an optionally substituted pyridine compound.
